# EUROPEAN PATENT SPECIFICATION

(11) **EP 1 591 088 B1**
(45) Date of publication and mention of the grant of the patent: **20.06.2007**
(21) Application number: 05103429.6
(22) Date of filing: 27.04.2005
(51) Int. Cl.: A61F 9/01

(54) **Refractive cornea ablation apparatus**
Refraktive Hornhautabtragungvorrichtung
Appareil d'ablation de la cornée refractive

(30) Priority: 27.04.2004 IT MI20040836
(43) Date of publication of application: 02.11.2005
(73) Proprietor: Ligi Tecnologie Medicali S.p.A., 74100 Taranto (IT)
(72) Inventor: D'Ippolito, Giuseppe, 74100 Taranto (IT); Lipari, Eugenio, 98021 Ali Terme (IT)
(74) Representative: Jorio, Paolo

(56) References cited:
- EP-A- 0 296 982
- WO-A-01/85045
- WO-A-95/27535
- DE-A1- 3 535 073
- US-A- 5 376 086

## Description

The present invention relates to a refractive cornea ablation apparatus.

More specifically, the present invention relates to a refractive cornea ablation apparatus employed in surgery to reshape the cornea to correct visual defects, as well as for cornea transplants.

Laser ablation (photoablation) of the cornea is now firmly established in ophthalmic surgery to remove portions of the cornea of a patient's eye.

The technique itself calls for an apparatus featuring a laser generator for emitting a laser beam, which travels through a focusing device and removes a portion of the cornea directly proportional to the power of the laser beam and to the exposure time of the selected cornea portion to the action of the laser beam.

The technique normally employs an excimer laser beam pulsated at a given frequency. The total number of pulses is calculated on the basis of the exposure time of a portion of the cornea to the action of the laser beam, so that, given the exposure time and the power of the laser beam, it is possible to calculate the depth of the cornea portion removed. In other words, the portion of the cornea struck by the beam sublimes and is eliminated easily by being in the gaseous state.

The focused laser beam is normally relatively small to permit intricate surgery and shape the cornea as closely as possible to the theoretically calculated shape.

Surgical ablation, however, must be performed extremely fast. For which purpose, a cornea ablation apparatus has been devised, in which the laser beam emitted by the generator is split into two beams, which are focused and directed separately onto separate outer surface areas of the cornea. The power of each of the resulting beams, however, is sufficient to remove a satisfactory amount of cornea tissue for each transmitted pulse. In other words, the resulting beams operate simultaneously on separate parts of the cornea, thus substantially reducing total surgery time.

The beam is split using semitransparent mirrors, which reflects a fraction of the energy of the incident beam, and lets the rest through.

This apparatus had drawbacks which make it practically impossible to split the beam accurately. Firstly, the balance between the reflected fraction and the transmitted fraction is greatly affected by the position of the mirror, which is a critical factor. That is, misalignment of even only a few degrees is sufficient to unbalance the resulting beams. Moreover, mirrors are subject to wear, so that, even if the mirror is positioned correctly, division of the incident energy between the two beams eventually undergoes changes anyway. Unbalance of the resulting beams is clearly harmful by resulting in removal of other than the calculated amount of tissue (more by one beam, and less by the other), so that the theoretical calculation of the ablation depth is not correct.

EP-A-0 296 982 discloses a refractive cornea ablation apparatus according to the preamble of claim 1.

It is an object of the present invention to provide a refractive cornea ablation apparatus of the type described above, designed to eliminate the drawbacks of the known art, and which, in particular, permits accurate division of the energy of a laser beam between different beams.

According to the present invention, there is provided a refractive cornea ablation apparatus as claimed in claim 1.

From one source, it is therefore possible to form two separate beams of identical characteristics.

In a preferred embodiment of the present invention, the apparatus is characterized by a homogenizing device located between said generator and said splitting device to distribute the energy of said first laser beam evenly over the respective cross section.

In a further preferred embodiment, the apparatus is characterized by an amplifying device located between said generator and said homogenizing device to impart said cross section to said first laser beam.

A preferred, non-limiting embodiment of the present invention will be described by way of example with reference to the accompanying drawings, in which:
Figure 1 shows a schematic side view, with parts removed for clarity, of an apparatus in accordance with the present invention;
Figure 2 shows a plan view, with parts removed for clarity, of the Figure 1 apparatus;
Figure 3 shows a larger-scale view in perspective of a detail of the Figure 1 apparatus.

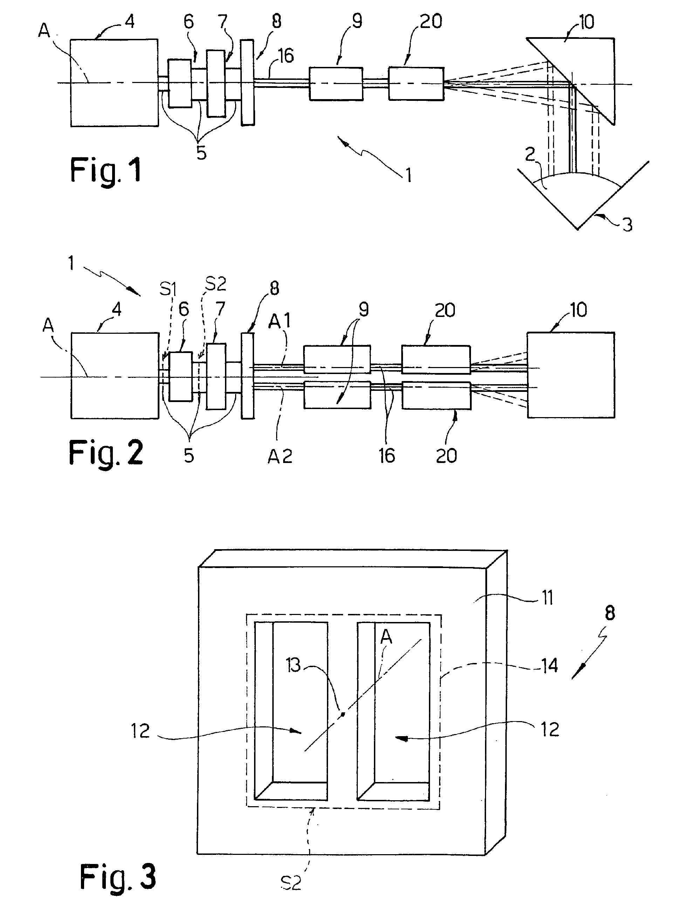

Number 1 in Figure 1 indicates as a whole an apparatus for refractive ablation of the cornea 2 of a patient's eye 3. The apparatus extends along an axis A of symmetry, along which are arranged in succession a generator 4 for generating a laser beam 5; an amplifying device 6 for amplifying beam 5; a homogenizing device 7 for homogenizing beam 5; a splitting device 8 for splitting beam 5; two optical focusing devices 9; two control devices 20, each comprising two independent known galvanometric mirrors (not shown); and a mirror 10.

Generator 4 pulsates the collimated laser beam 5, which extends along axis A with a substantially rectangular cross section S1; amplifying device 6 then amplifies beam 5 to a cross section S2 larger than S1; and homogenizing device 7 modifies beam 5 to evenly spread energy per unit of area over cross section S2.

With reference to Figure 3, splitting device 8 comprises a plate 11, in which two identical rectangular openings 12 are formed; plate 11 has a centre of gravity 13 located along axis A; and openings 12 are located symmetrically on opposite sides of axis A, so as to interfere with beam 5. In other words, rectangular cross section S2 defines on plate 11 a rectangle 14, whose centre is defined by centre of gravity 13, and which surrounds openings 12.

Part of beam 5 is shielded by plate 11, while two separate identical portions travel through respective openings 12 to substantially define two laser beams 16, which extend along respective axes A1, A2 parallel to and located symmetrically on opposite sides of axis A.

With reference to Figure 2, the two beams 16 impinge respective identical focusing devices 9, which are located symmetrically along respective axes A1 and A2 and are therefore barycentric. Beams 16 are oriented separately and independently by control devices 20 to sweep respective surface portions of cornea 2 of eye 3 following given paths, according to a known method not shown in detail (the dash lines in Figures 1 and 2 show different angles of beams 16 issuing from control devices 20). With reference to Figure 1, each beam 16 is then diverted by mirror 10 onto the patient's eye 3.

To minimize energy dissipation, amplifying device 6 is regulated so that cross section S2 is fitted around openings 12.

Though the embodiment described refers to a plate 11 comprising two openings 12, it is understood that the present invention also applies to plates comprising a number of openings for generating a number of parallel beams, each associated with a respective barycentric focusing device.

## Claims

1. A refractive cornea ablation apparatus comprising a laser generator for generating a first laser beam (5), and a splitting device (8) for producing at least two separate second laser beams (16) from said first laser beam (5); wherein said splitting device (8) comprises a plate (11) having at least two identical openings (12), through which respective separate portions of the first laser beam (5), defining said second laser beams (16), travel ; the apparatus comprising an optical focusing device (9) for each second laser beam (16);
**characterized by** comprising, for each second laser beam (16), an optical control device (20) for orienting said second laser beams (16) separately and independently.

2. An apparatus as claimed in Claim 1, **characterized by** extending along a longitudinal first axis (A), along which the first laser beam (5) extends; said openings (12) being located symmetrically on opposite sides of said first axis (A).

3. An apparatus as claimed in Claim 2, **characterized in that** said second laser beams (16) extend along respective second axes (A1, A2) parallel to the first axis (A).

4. An apparatus as claimed in any one of the foregoing Claims, **characterized in that** said first laser beam (5) has a cross section (S2) defining on said plate (11) a plane figure (14) circumscribing said openings (12).

5. An apparatus as claimed in Claim 4, **characterized in that** said plane figure is a rectangle (14).

6. An apparatus as claimed in Claim 3, **characterized in that** a homogenizing device (7) is located between said generator (4) and said splitting device (8) to distribute the energy of said first laser beam (5) evenly over a respective cross section (S2).

7. An apparatus as claimed in Claim 6, **characterized in that** an amplifying device (6) is located between said generator (4) and said homogenizing device (7) to impart said cross section (S2) to said first laser beam (5).

## Patentansprüche

1. Vorrichtung zur refraktiven Hornhautentfernung, einen Laser-Generator zum Erzeugen eines ersten Laserstrahls (5) und ein Aufteilungsgerät (8) umfassend, um zumindest zwei getrennte zweite Laserstrahlen (16) aus dem ersten Laserstrahl (5) zu erzeugen; wobei das Aufteilungsgerät (8) eine Platte (11) mit mindestens zwei identischen Öffnungen (12) umfasst, durch die die entsprechenden einzelnen Teile des ersten Laserstrahls (5), die die zweiten Laserstrahlen (16) definieren, durchgehen; wobei der Apparat ein optisches Fokussiergerät (9) für jeden zweiten Laserstrahl (16) umfasst;
**dadurch gekennzeichnet, dass**
dieses für jeden zweiten Laserstrahl (16) ein optisches Steuergerät (20) zur Ausrichtung der zweiten Laserstrahlen (16) in eigenständiger und unabhängiger Weise umfasst.

2. Vorrichtung gemäß Anspruch 1, durch die Ausdehnung entlang einer ersten Längsachse (A) kennzeichnet, entlang der der erste Laserstrahl (5) verläuft; wobei sich die Öffnungen (12) symmetrisch auf einander gegenüber liegenden Seiten der ersten Achse (a) befinden.

3. Vorrichtung gemäß Anspruch 2, **dadurch gekennzeichnet, dass** die zweiten Laserstrahlen (16) entlang der entsprechenden zweiten Achsen (A1, A2) parallel zur ersten Achse (A) verlaufen.

4. Vorrichtung gemäß einem der obigen Ansprüche, **dadurch gekennzeichnet, dass** der erste Laserstrahl (5) einen Querschnitt (S2) aufweist, der auf der Platte (11) eine ebene Figur (14) definiert, die die Öffnungen (12) umgrenzt.

5. Vorrichtung gemäß Anspruch 4, **dadurch gekennzeichnet, dass** die ebene Figur ein Rechteck (14) ist.

6. Vorrichtung gemäß Anspruch 3, **dadurch gekennzeichnet, dass** sich ein Homogenisierungsgerät (7) zwischen dem ersten Generator (4) und dem Aufteilungsgerät (8) befindet, um die Energie des ersten Laserstrahls (5) gleichmäßig über den entsprechenden Querschnitt (S2) zu verteilen.

7. Vorrichtung gemäß Anspruch 6, **dadurch gekennzeichnet, dass** sich ein Verstärkungsgerät (6) zwischen dem Generator (4) und dem Homogenisierungsgerät (7) befindet, um den Querschnitt (S2) an den ersten Laserstrahl weiterzugeben.

## Revendications

1. Appareil d'ablation de la cornée réfractive comprenant un générateur de laser destiné à générer un premier faisceau laser (5), et un dispositif de séparation (8) destiné à produire au moins deux seconds faisceaux laser séparés (16) à partir dudit premier faisceau laser (5) ; dans lequel ledit dispositif de séparation (8) comprend une plaque (11) ayant au moins deux ouvertures identiques (12), dans lesquelles des parties séparées respectives du premier faisceau laser (5), définissant lesdits seconds faisceaux laser (16) se déplacent ; l'appareil comprenant un dispositif de mise au point optique (9) destiné à chaque second faisceau laser (16) ; l'appareil étant **caractérisé en ce qu'**il comprend, pour chaque second faisceau laser (16), un dispositif de commande optique (20) destiné à orienter lesdits seconds faisceaux laser (16) de manière séparée et indépendante.

2. Appareil selon la revendication 1, **caractérisé en ce qu'**il s'étend le long d'un premier axe longitudinal (A), le long duquel le premier faisceau laser (5) s'étend ; lesdites ouvertures (12) étant situées symétriquement sur les côtés opposés dudit premier axe (A).

3. Appareil selon la revendication 2, **caractérisé en ce que** lesdits seconds faisceaux laser (16) s'étendent le long de seconds axes respectifs (A1, A2) parallèles au premier axe (A).

4. Appareil selon l'une quelconque des revendications précédentes, **caractérisé en ce que** ledit premier faisceau laser (5) possède une section transversale (S2) définissant, sur ladite plaque (11), une figure plane (14) entourant lesdites ouvertures (12).

5. Appareil selon la revendication 4, **caractérisé en ce que** ladite figure plane est un rectangle (14).

6. Appareil selon la revendication 3, **caractérisé en ce qu'**un dispositif d'homogénéisation (7) est situé entre ledit générateur (4) et ledit dispositif de séparation (8) afin de répartir l'énergie dudit premier faisceau laser (5) de manière uniforme sur une section transversale respective (S2).

7. Appareil selon la revendication 6, **caractérisé en ce qu'**un dispositif d'amplification (6) est situé entre ledit générateur (4) et ledit dispositif d'homogénéisation (7) afin de transmettre ladite section transversale (S2) audit premier faisceau laser (5).
